# EUROPEAN PATENT APPLICATION

(11) **EP 4 740 946 A1**
(43) Date of publication of application: **13.05.2026**
(21) Application number: 24835424.3
(22) Date of filing: 05.07.2024
(51) Int. Cl.: A61K 31/496, A61P 13/08, A61P 35/00, A61P 35/04

(54) **METHOD FOR TREATING PROSTATE CANCER**

(30) Priority: 06.07.2023 CN 202310825049
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: WANG, Wenliang, Shanghai 201210 (CN)
(74) Representative: Regimbeau
(86) International application number: PCT/CN2024/103772
(87) International publication number: WO 2025/007946

(57) **Abstract**

A method for treating prostate cancer; specifically, the use of a compound of formula I or a pharmaceutically acceptable salt thereof in the preparation of a drug for treating metastatic castration-resistant prostate cancer.

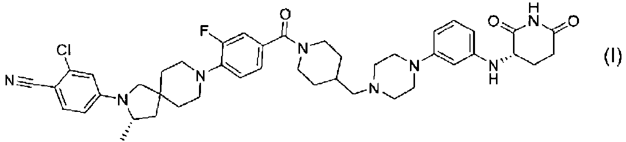

## Description

### Technical Field

The present application relates to the field of medicine, and includes the use of a compound of formula I or a pharmaceutically acceptable salt thereof in the preparation of a drug for treating prostate cancer.

### Background Art

Androgen receptor (AR) is a ligand-dependent transcriptional regulatory protein that belongs to the nuclear receptor superfamily and is mainly found in the nucleus. AR that is not bound to the ligand binds to heat shock protein (HSP); however, after AR binds to the ligand, it undergoes a conformational change and dissociates from the HSP, and exhibits increased affinity for DNA (the activation of AR). Activated AR binds in dimer form to a specific DNA sequence in the nucleus (i.e., androgen response element, ARE) and interacts with other transcription factors, thereby regulating the expression of relevant genes and producing biological effects. Studies have shown that abnormalities in the AR signalling pathway are closely related to the development and progression of diseases such as prostate cancer, benign prostatic hyperplasia, Kennedy's disease, male infertility, androgen insensitivity syndrome, and male breast cancer.

Prostate cancer is one of the most common malignancies. According to statistics, in 2018, there were nearly 1.3 million new cases and 359,000 deaths worldwide, accounting for 13.5% of the incidence of malignancies in men, ranking second, and 6.7% of the mortality of malignancies in men, ranking fifth. At present, the incidence of prostate cancer is increasing annually, and making it a major cause of death among male cancer patients. A number of AR antagonists (e.g., the first generation AR inhibitor bicalutamide, and the second generation AR inhibitors enzalutamide and rezvilutamide) have been approved for marketing, successfully used in the treatment of hormone-sensitive and castration-resistant prostate cancer, and have become a major treatment for prostate cancer. Although the development and application of the new AR-targeted drugs have significantly improved the prognosis of patients with prostate cancer, particularly providing alternative treatment options other than chemotherapy for patients with mCRPC, patients still may develop resistance to the novel AR-targeted drugs.

### Summary of the Invention

The present disclosure provides a compound of formula I or a pharmaceutically acceptable salt thereof for treating metastatic castration-resistant prostate cancer,

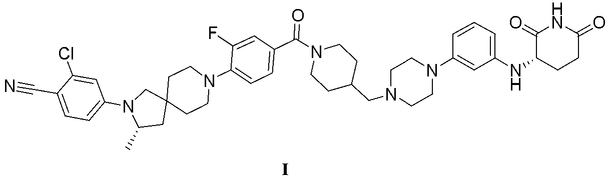

In certain embodiments, the present disclosure provides a method of treating metastatic castration-resistant prostate cancer, the method comprising administering to a patient a therapeutically effective amount of the compound of formula I or the pharmaceutically acceptable salt thereof.

In certain embodiments, the present disclosure provides a method of treating metastatic castration-resistant prostate cancer, the method comprising administering to a patient a therapeutically effective amount of the compound of formula I or the pharmaceutically acceptable salt thereof after a meal.

The present disclosure also provides a method of treating metastatic castration-resistant prostate cancer, the method comprising administering to a patient a therapeutically effective amount of the compound of formula I or the pharmaceutically acceptable salt thereof and a therapeutically effective amount of a CYP3A4 enzyme substrate.

In certain embodiments, the method for treating metastatic castration-resistant prostate cancer described in the present disclosure, which comprises administering to the patient a therapeutically effective amount of the compound of formula I or the pharmaceutically acceptable salt thereof and a therapeutically effective amount of a CYP3A4 enzyme substrate, does not require adjustment of the administration dose of the compound of formula I or the pharmaceutically acceptable salt thereof.

In certain embodiments, the CYP3A4 enzyme substrate described in the present disclosure is midazolam.

In certain embodiments, the metastatic castration-resistant prostate cancer described in the present disclosure is metastatic castration-resistant prostate cancer that has been treated with medical castration or surgical castration.

In certain embodiments, the metastatic castration-resistant prostate cancer described in the present disclosure is selected from metastatic castration-resistant prostate cancer that has been treated with continuous luteinizing hormone-releasing hormone analogue (LHRHA) (medical castration), or has previously undergone bilateral orchidectomy (surgical castration), or has maintained effective LHRHA therapy without bilateral orchidectomy.

In certain embodiments, the patient with metastatic castration-resistant prostate cancer described in the present disclosure has a castrate level of testosterone (≦50 ng/dL or 1.73 nmol/L).

In certain embodiments, the metastatic castration-resistant prostate cancer described in the present disclosure is metastatic castration-resistant prostate cancer that has failed at least one prior novel endocrine drug therapy.

In certain embodiments, the metastatic castration-resistant prostate cancer described in the present disclosure is metastatic castration-resistant prostate cancer that has failed at least one prior endocrine drug therapy.

In certain embodiments, the novel endocrine drug described in the present disclosure is selected from abiraterone, enzalutamide, apalutamide, rezvilutamide or darolutamide.

In certain embodiments, the endocrine drug described in the present disclosure is selected from abiraterone, enzalutamide, apalutamide, rezvilutamide or darolutamide.

Treatment failure described in the present disclosure refers to disease progression during treatment.

In certain embodiments, the metastatic castration-resistant prostate cancer described in the present disclosure is metastatic castration-resistant prostate cancer that has been previously treated with at least one chemotherapeutic drug and has progressed during chemotherapy or within 6 months after chemotherapy, or is unsuitable for or intolerant to a chemotherapeutic drug.

In certain embodiments, the chemotherapeutic drug described in the present disclosure is selected from a taxane-based chemotherapeutic drug, preferably the taxane-based chemotherapeutic drug is selected from docetaxel.

The disease progression described in the present disclosure is defined as the occurrence of one or more of the following three events in a subject while undergoing castration therapy:
(1) PSA progression, including progression to CRPC during HSPC treatment (defined as a PSA value > 1 ng/mL at a castrate level, with the PSA level increased by > 50% compared to baseline for 2 consecutive times with an interval of at least 1 week), and PSA progression during CRPC treatment (according to PCWG3 criteria); where for patients treated with flutamide or bicalutamide, PSA progression must also occur after drug withdrawal (for ≧ 4 weeks and ≧ 6 weeks, respectively);
(2) disease progression as defined in RECIST 1.1;
(3) skeletal disease progression as defined in PCWG3 criteria, i.e., the detection of ≧ 2 new lesions on bone scan.

In certain embodiments, the compound of formula I or the pharmaceutically acceptable salt thereof described in the present disclosure is administered at a dose of 10 mg-1000 mg, preferably at a dose of 20 mg -500 mg, and more preferably at a dose of 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg, 480 mg, 490 mg or 500 mg.

In certain embodiments, the compound of formula I or the pharmaceutically acceptable salt thereof described in the present disclosure is administered at a dose of 30 mg, 90 mg, 180 mg, 360 mg, 540 mg or 720 mg.

In certain embodiments, the compound of formula I or the pharmaceutically acceptable salt thereof described in the present disclosure is administered at a dose selected from 10 mg-300 mg; preferably 10 mg-200 mg; more preferably 10 mg-100 mg; and more preferably 10 mg-50 mg.

In certain embodiments, the compound of formula I or the pharmaceutically acceptable salt thereof described in the present disclosure has a unit dose of 10 mg-300 mg; preferably 10 mg-200 mg; more preferably 10 mg-100 mg; and more preferably 10 mg-50 mg.

In certain embodiments, the compound of formula I or the pharmaceutically acceptable salt thereof described in the present disclosure has a unit dose of 10 mg-1000 mg, preferably a unit dose of 20 mg -500 mg, and preferably a unit dose of 15 mg, 30 mg, 60 mg, 90 mg, 180 mg, 360 mg, 540 mg or 720 mg.

In certain embodiments, the administration dose or the unit dose described in the present disclosure is calculated based on the weight of the compound of formula I.

In certain embodiments, the frequency of administration of the compound of formula I or the pharmaceutically acceptable salt thereof described in the present disclosure is selected from three times a day, twice a day, once a day, once every two days, once every three days, once every four days, once every five days, and once a week.

In certain embodiments, the subject for administration of the compound of formula I or the pharmaceutically acceptable salt thereof described in the present disclosure is a human.

In certain embodiments, the compound of formula I or the pharmaceutically acceptable salt thereof described in the present disclosure is administered in the form of a pharmaceutical composition comprising the compound of formula I or the pharmaceutically acceptable salt thereof and one or more pharmaceutically acceptable carriers, excipients or diluents.

"Administering to a patient a therapeutically effective amount of the compound of formula I or the pharmaceutically acceptable salt thereof and a therapeutically effective amount of a CYP3A4 enzyme substrate" described in the present disclosure refers to administering the compound of formula I or the pharmaceutically acceptable salt thereof and at least one CYP3A4 enzyme substrate within a period of time. The time period may be within an administration cycle, optionally within 72 hours, within 36 hours, within 48 hours, within 24 hours, within 20 hours, within 18 hours, within 16 hours, within 12 hours, within 10 hours, within 8 hours, within 6 hours, within 4 hours, within 2 hours, within 1 hour, or within 0.5 hours, or the compound of formula I or the pharmaceutically acceptable salt thereof and at least one CYP3A4 enzyme substrate are simultaneously administrated. Preferably, the time period is within 24 hours, 20 hours, 18 hours, 16 hours, 12 hours, 10 hours, 8 hours, 6 hours, 4 hours, 2 hours, 1 hour, or 0.5 hours, or the compound of formula I or the pharmaceutically acceptable salt thereof and at least one CYP3A4 enzyme substrate are simultaneously administrated.

### Detailed Description of Embodiments

### Example 1 Phase I clinical study of the compound of formula I for safety, tolerability, pharmacokinetic and efficacy in subjects with advanced metastatic castration-resistant prostate cancer

### 1. Study drugs

Test drug 1: The compound of formula I (15 mg/tablet and 60 mg/tablet) was prepared with reference to WO 2023093845 A and produced and supplied by Jiangsu Hengrui Pharmaceuticals Co., Ltd.

Test drug 2: Midazolam injection, 1 ml: 5 mg, Jiangsu Nhwa Pharmaceutical Co., Ltd.

### 2. Study objective

### Primary study objective:

To evaluate the safety and tolerability of the compound of formula I in subjects with advanced metastatic castration-resistant prostate cancer (mCRPC).

### Secondary study objectives:

To evaluate the pharmacokinetic (PK) profile of the compound of formula I and major metabolites thereof in subjects with advanced mCRPC;
to evaluate the effect of the compound of formula I on the pharmacokinetic of midazolam (a CYP3A4 enzyme substrate) following multiple administrations;
to evaluate the initial efficacy of the compound of formula I in subjects with advanced mCRPC.

### 3. Study endpoints

### Primary study endpoints

- Incidence of dose limiting toxicity (DLT)
- MTD (if available) and RP2D;

### Secondary study endpoints

- Incidence, severity (graded according to CTCAE v5.0), and correlation with the study drug of adverse events (AEs) and serious adverse events (SAEs), also including abnormalities in vital signs, electrocardiogram, and laboratory tests;
- PK parameters of the compound of formula I after single/multiple administrations, including but not limited to:
   single administration parameters: peak plasma concentration (Cmax), time to peak (Tmax), area under the concentration-time curve from time zero to the last detectable concentration time point t (AUC0-t), area under the concentration-time curve from time zero to infinity (AUC0-inf) (if applicable), half-life (t1/2) (if applicable), apparent volume of distribution (Vz/F) (if applicable), and apparent clearance (CL/F) (if applicable);
   multiple administration parameters: steady-state peak concentration (Cmax, ss), time to peak (Tmax, ss), steady-state trough concentration (Cmin, ss), area under the steady-state plasma concentration-time curve (AUCss), and drug accumulation ratio (Rac).
- Pharmacokinetic (PK) of midazolam: pharmacokinetic parameters of midazolam in patients after administration of a microdose of midazolam before (D-1) and after (D15) multiple oral administrations of the compound of formula I, including: Tmax, Cmax, AUC0-t, AUCO-∞, t1/2, CL/F and Vz/F.
- Efficacy endpoints: objective response rate (ORR), disease control rate (DCR), duration of response (DoR), radiographic progression-free survival (rPFS), PSA response rate at week 12, proportions of subjects with a decrease in PSA of ≧50% and ≧30% from baseline throughout study treatment, time to PSA progression, and overall survival (OS).

### 4. Inclusion criteria

Subjects must meet all of the following inclusion criteria to be enrolled in this study:
1) voluntary participation in this clinical study, understanding the study procedures, and being able to sign the written informed consent form;
2) aged 18-80 years old (inclusive), male;
3) Eastern Cooperative Oncology Group (ECOG) performance status score of 0 -1;
4) predicted survival time of ≥ 12 weeks;
5) prostatic adenocarcinoma confirmed by histopathological or cytological examination, without a diagnosis of neuroendocrine carcinoma or small cell carcinoma;
6) continuous luteinizing hormone-releasing hormone analogue (LHRHA) therapy (medical castration), or previous bilateral orchidectomy (surgical castration); where subjects who have not undergone bilateral orchidectomy must plan to maintain effective LHRHA therapy throughout the study;
7) testosterone at castrate level (≦50 ng/dL or 1.73 nmol/L) at screening;
8) previous treatment failure (defined as disease progression during treatment, the definition of disease progression being the same as that in inclusion criterion 10) with at least one novel endocrine drug (e.g., abiraterone, and enzalutamide);
9) patients who has previously received at least one taxane-based chemotherapeutic drug (e.g., docetaxel) and has progressed during chemotherapy or within 6 months after chemotherapy (the definition of disease progression being the same as that in inclusion criterion 10), or are unsuitable for or intolerant to chemotherapeutic drugs;
10) disease progression, which is defined as the occurrence of one or more of the following three events in a subject while undergoing castration therapy: (1) PSA progression, including progression to CRPC during HSPC treatment (defined as a PSA value > 1 ng/mL at a castrate level, with the PSA level increased by > 50% compared to baseline for 2 consecutive times with an interval of at least 1 week), and PSA progression during CRPC treatment (according to PCWG3 criteria); where for patients treated with flutamide or bicalutamide, PSA progression must also occur after drug withdrawal (for ≧ 4 weeks and ≧ 6 weeks, respectively); (2) disease progression as defined in RECIST 1.1; (3) skeletal disease progression as defined in PCWG3 criteria, i.e., the detection of ≧2 new lesions on bone scan;
11) having metastatic lesions confirmed by imaging examinations including CT/MRI or radionuclide bone scan (99mTc);
12) must providing sufficient blood samples during the screening period for gene mutation testing; where it is recommended that tumour tissue samples (archived samples or fresh biopsy samples are acceptable; fresh samples or archived samples within 1 year are preferred) are also provided;
13) sufficient organ function as defined by the following laboratory tests:
   - routine blood tests (no blood transfusion or blood products administration, and no G-CSF or other hematopoietic stimulating factors for correction within 14 days prior to the first administration): absolute neutrophil count (ANC) ≥ 1.5 × 109/L; platelet count (PLT) ≥ 100 × 109/L; hemoglobin (Hb) ≥ 90 g/L;
   - hepatic function tests: total bilirubin (TBIL) ≤ 1.5 × upper limit of normal (ULN) (≤ 2 × ULN if liver metastasis occurs); serum transaminase ALT and/or AST ≤ 3 × ULN (≤ 5 × ULN if liver metastasis occurs);
   - renal function tests: serum creatinine (Cr) ≤ 1.5 × ULN; where if urine routine shows urine protein ≥++, 24-hour urine protein excretion must be confirmed to be ≤ 1.0 g;
   - electrocardiogram tests: QT interval corrected by Fridericia's formula (QTcF) being normal (≤ 470 ms for males);
   - echocardiogram tests: left ventricular ejection fraction (LVEF) ≥ 50%;
   - coagulation function tests: APTT ≤ 1.5 × ULN, while INR or PT ≤ 1.5 × ULN.
14) male subjects whose partners are women of child-bearing age and who must agree to refrain from sperm donation and use appropriate contraceptive measures from the time of signing the informed consent form until 3 months after the last administration of the test drug.

### 4. Exclusion criteria

Subjects will be excluded from this study if they meet any one of the following criteria:
1) previous treatment with any AR degrader;
2) planned receipt of any other anti-tumour treatment during this study;
3) receiving chemotherapy, targeted therapy, immunotherapy, live attenuated vaccination, radiotherapy, surgical treatment, etc. less than 4 weeks before the start of study treatment (for small-molecule targeted drugs, 7 days or 5 times the drug half-life before the first study drug administration, whichever is longer; the washout period for bicalutamide being 6 weeks); participating in another clinical study 4 weeks before the start of study treatment (calculated from the time of last use of test drugs or devices);
4) damage caused by any previous anti-tumour treatments that have not resolved to ≤ grade 1 or the criteria specified in this study (graded according to NCI-CTCAE 5.0; excluding alopecia and other adverse events deemed tolerable by the investigator);
5) previous use of drugs that affect the activity of metabolic enzyme CYP3A, with a washout period from the end of drug use to the first administration of this study shorter than 5 half-lives of the drug;
6) patients who have participated in QT/QTc studies and have been treated with any drug that has a risk of QT/QTc interval prolongation or torsades de pointes (TdP) within 4 weeks before the first administration, previously had congenital prolonged QT syndrome or family history of prolonged QT interval, have an implantable pacemaker or automatic implantable cardioverter-defibrillator, or have uncorrectable electrolyte disturbances or other factors that interfere with QT/QTc studies;
7) subjects who have poorly controlled hypertension (systolic blood pressure > 150 mmHg and/or diastolic blood pressure > 100 mmHg under regular antihypertensive treatment), or previously have a history of hypertensive crisis or hypertensive encephalopathy;
8) known central nervous system metastasis or meningeal metastasis of tumour, or a history of primary central nervous system tumours in the subject;
9) severe bone injury caused by bone metastasis of tumour, as determined by the investigator, including poorly controlled severe bone pain, and pathological fractures, spinal cord compression, etc. at key sites that have occurred within the past 6 months or are expected to occur in the near future;
10) any one of multiple factors that affect oral drug administration (e.g., inability to swallow, chronic diarrhoea and intestinal obstruction), or active gastrointestinal disease or other diseases that may lead to factors significantly affecting drug absorption, distribution, metabolism, or excretion;
11) known allergy or intolerance to the compound of formula I or auxiliary materials thereof;
12) active cardiac disease within 6 months before the first administration, including: severe/unstable angina, myocardial infarction, symptomatic congestive heart failure, and ventricular arrhythmias requiring drug treatment;
13) other malignancies within 5 years before the first administration (excluding carcinoma in situ that has achieved complete remission and malignancies that are assessed by the investigator to be slowly progressive);
14) active hepatitis B (HBsAg positive with HBV DNA ≥ 500 IU/ml), hepatitis C (hepatitis C antibody positive with HCV RNA above the lower limit of detection of the analytical method); or patients with severe infections that require antibiotics, antivirals, or antifungals;
15) history of immunodeficiency (including positive HIV test, and other acquired or congenital immunodeficiency diseases) or organ transplantation;
16) subjects with other factors, as determined by the investigator, that may affect study results or lead to the termination of this study, such as alcoholism, substance abuse, other serious diseases (including mental diseases) requiring concurrent treatment, severely abnormal laboratory test values, family or social factors, or other conditions that may affect subject safety or the collection of trial data.

### 5. Mode of administration

Tablets were orally administered in preset dose group of 30 mg, 90 mg, 180 mg, 360 mg, 540 mg or 720 mg, with one treatment cycle every 28 days. Administration was continuous until disease progression or the occurrence of unacceptable toxicity. Tablets were orally administered once daily, within 30 minutes after breakfast each morning, for consecutive administration. If intensive PK blood sampling was scheduled on a given day, the subject was forbidden to drink water 1 h and fasted 4 h before and after drug administration.

In drug interaction (DDI) assessment phase, tablets were orally administered once daily, within 30 minutes after breakfast each morning, for consecutive administration. Midazolam (100 µg) was administered once within 30 min after breakfast in the morning on day -1 and on day 15. Midazolam and the test drug were administered simultaneously on day 15 (± 2 min).

### 6. Preliminary results

### 6.1 Clinical pharmacology

There were a total of 9 patients in the food effect study. For the 180 mg administration under fasting conditions, the mean half-life (± standard deviation) was 40.8 ± 4.37 h, the geometric mean (% geometric coefficient of variation) of apparent volume of distribution was 1260 (90.7) L, the geometric mean (% geometric coefficient of variation) of clearance was 21.5 (83.4) L/h, and the median time to peak was 3 h.

Postprandial absorption was greater than that observed in the fasting state, with a 2.54-fold higher in AUC, a 3.06-fold higher in Cmax, and the time to peak delayed by 3 hours (about 6 h).

### 6.2 Clinical safety

A total of 26 tumour subjects were enrolled in this study, with 13 subjects in the dose escalation phase. There were 1, 4, 4, 3, and 1 subject(s) in 30 mg, 90 mg, 180 mg, 360 mg, and 540 mg dose groups, respectively. All these subjects have completed dose-limiting toxicity (DLT) observation, and no DLT was observed. One subject was enrolled in the 540 mg dose group and remained in DLT observation, and no dose-limiting toxicity (DLT) was observed. There were 13 subjects in the dose expansion phase, and all were in the 180 mg dose group.

A total of 9 (34.6%) subjects experienced TEAEs during the study, and the TEAEs reported in at least 2 subjects include: anaemia, hypercholesterolemia, increased serum creatinine, and rash (two cases each). Three (11.5%) subjects experienced grade 3 TEAEs, including decreased lymphocyte count, increased blood pressure, decreased neutrophil count, and decreased white blood cell count, 1 case each.

A total of 8 (30.8%) subjects experienced AEs associated with formula I during the study and the AEs reported in at least 2 subjects include: anaemia, hypercholesterolemia and increased serum creatinine (2 cases each). One (3.8%) subject experienced grade 3 TRAE, including decreased neutrophil count, and decreased white blood cell count.

No grade 4/5 AE occurred during the study.

### 6.3 Clinical effectiveness

A total of 17 subjects in the study received at least one post-baseline PSA test, with 1, 4, 9, and 3 subjects in 30 mg, 90 mg, 180 mg, and 360 mg dose groups, respectively. 8 of 17 subjects evaluable for PSA response experienced a PSA decrease. In particular, 6 (66.7%) of the 9 subjects in the 180 mg dose group experienced a PSA decrease, with 3 subjects achieving PSA30 (a reduction in serum PSA level of ≧ 30% from baseline during treatment), including 1 subject achieving PSA90 (a reduction in serum PSA level of ≧ 90% from baseline during treatment).

## Claims

1. Use of a compound of formula I or a pharmaceutically acceptable salt thereof in the preparation of a drug for treating metastatic castration-resistant prostate cancer

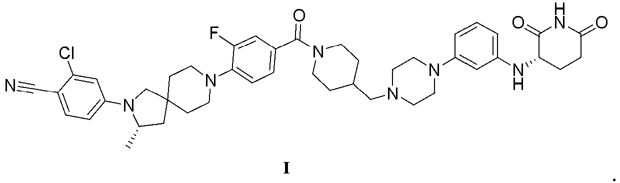

2. The use according to claim 1, **characterised in that** the metastatic castration-resistant prostate cancer is metastatic castration-resistant prostate cancer that has been treated with medical castration or surgical castration, or has maintained effective LHRHA therapy without bilateral orchidectomy.

3. The use according to claim 1, **characterised in that** the metastatic castration-resistant prostate cancer is metastatic castration-resistant prostate cancer that has failed at least one prior endocrine drug therapy; and the endocrine drug is preferably abiraterone, enzalutamide, apalutamide, rezvilutamide or darolutamide.

4. The use according to claim 1, **characterised in that** the metastatic castration-resistant prostate cancer is metastatic castration-resistant prostate cancer that has been previously treated with at least one chemotherapeutic drug and has progressed during chemotherapy or within 6 months after chemotherapy, or is unsuitable for or intolerant to a chemotherapeutic drug; the chemotherapeutic drug is preferably a taxane-based chemotherapeutic drug, and more preferably docetaxel.

5. The use according to claim 1, **characterised in that** the subject for administration of the compound of formula I or the pharmaceutically acceptable salt thereof is a human.

6. The use according to claim 1, **characterised in that** the compound of formula I or the pharmaceutically acceptable salt thereof is administered at a dose of 10 mg-1000 mg.

7. The use according to claim 1, **characterised in that** the compound of formula I or the pharmaceutically acceptable salt thereof is administered at a dose of 30 mg, 90 mg, 180 mg, 360 mg, 540 mg or 720 mg.

8. The use according to claim 1, **characterised in that** the compound of formula I or the pharmaceutically acceptable salt thereof has a unit dose of 10 mg-300 mg.
